# EUROPÄISCHE PATENTSCHRIFT

(11) **EP 2 802 272 B1**
(45) Veröffentlichungstag und Bekanntmachung des Hinweises auf die Patenterteilung: **06.03.2019**
(21) Anmeldenummer: 12809660.9
(22) Anmeldetag: 07.12.2012
(51) Int. Cl.: A61B 17/02

(54) **CHIRURGISCHE RETRAKTIONSVORRICHTUNG**
SURGICAL RETRACTOR
DISPOSITIF DE RÉTRACTION CHIRURGICAL

(30) Priorität: 13.01.2012 DE 102012100284
(43) Veröffentlichungstag der Anmeldung: 19.11.2014
(73) Patentinhaber: Aesculap AG, 78532 Tuttlingen (DE)
(72) Erfinder: BECK, Thomas, 78591 Durchhausen (DE); KLEINE, Peter, 63263 Neu Isenburg (DE); WEISSHAUPT, Dieter, 78194 Immendingen (DE); MORALES, Pedro, 78532 Tuttlingen (DE); VOGTHERR, Robert, 78532 Tuttlingen (DE); ELISCH, Andreas, 78713 Schramberg (DE)
(74) Vertreter: Hoeger, Stellrecht & Partner Patentanwälte mbB
(86) Internationale Anmeldenummer: PCT/EP2012/074802
(87) Internationale Veröffentlichungsnummer: WO 2013/104473

(56) Entgegenhaltungen:
- DE-U1-202011 051 999
- US-A- 2 670 731
- US-A- 4 852 552
- US-A1- 2008 139 879
- US-A1- 2009 259 107

## Beschreibung

Die Erfindung betrifft eine chirurgische Retraktionsvorrichtung, insbesondere zum Spreizen eines durchtrennten Sternums, mit einer Haltevorrichtung sowie zwei Spreizarmen, wobei die Spreizarme mit einem Ende an der Haltevorrichtung in einem in einer Spreizebene veränderlichen Abstand voneinander gehalten sind. An jedem der Spreizarme sind ein oder mehrere Rückhalteelemente zum Rückhalten von Knochenmaterial, beispielsweise des durchtrennten Sternums, angeordnet.

Chirurgische Retraktionsvorrichtungen der eingangs genannten Art werden insbesondere bei Operationen am Herzen verwendet, um einen entsprechenden Zugang zu halten. Dabei wird das Sternum in seiner Längsrichtung mittig durchtrennt und die Sternumhälften werden zusammen mit den daran anschließenden Rippen mittels der Retraktionsvorrichtung nach beiden Seiten zurückgedrängt, d.h. retrahiert.

Aufgrund der Rippenform und der Rippenanordnung ist die beobachtete Steifigkeit am cranialseitigen Ende der Sternumhälften deutlich höher als am caudal gelegenen Ende.

Aufgrund dieser unterschiedlichen Steifigkeit treten insbesondere bei herkömmlichen Retraktionsvorrichtungen, bei denen die erzielten Öffnungsweiten des Sternums cranial und caudal gleich sind, ohne zusätzliche Nachstell- oder Justiermöglichkeit entlang der vertikalen Achse des Sternums ungünstige Kräfteverhältnisse auf.

Insbesondere bei den cranial gelegenen Rippen treten aufgrund der dort wesentlich höheren Steifigkeit höhere Kräfte auf als bei den caudal gelegenen Rippen. Dies resultiert wiederum in erhöhten Spannungen in diesen Rippen, wodurch postoperativ erhöhte Schmerzen auftreten können.

Die Schmerzen resultieren vor allem dann, wenn eine cranial gelegene Rippe den im Bereich der Schulter liegenden Unterarm-Plexus (Plexus brachialis), durch ihre Verformung oder gar Absplitterung von Teilen in diesem Bereich schädigt.

Im Extremfall kann es sogar zum Bruch einer cranial gelegenen Rippe kommen. Da diese Rippen unterhalb des Schlüsselbeins liegen, werden Rippenbrüche im normalen Röntgenbild nicht erkannt. Sie kommen aber laut neueren Studien häufiger vor als bisher vermutet.

Aus der DE 20 2011 051 999 U1 ist ein chirurgisches Retraktionssystem bekannt, bei dem zwei einteilige Spreizarme in einem in einer Retraktionsrichtung veränderlichen Abstand voneinander an einer Haltevorrichtung gehalten sind. An den Spreizarmen sind jeweils zwei Rückhalteelemente gehalten.

In der US 2008/0138979 A1 ist eine Haltevorrichtung für Gewebe beschrieben, bei der Rückhalteelemente mit einer federelastischen Lagerung an der Vorrichtung gehalten sind.

Das U.S.-Patent US 2,670,731 schlägt ein chirurgisches Retraktionssystem vor, bei dem die Spreizarme in zwei Abschnitte unterteilt sind, wobei jeweils ein erster Abschnitt an einer Haltevorrichtung gehalten ist und ein zweiter Abschnitt schwenkbar am ersten Abschnitt gelagert ist. Am zweiten Abschnitt ist jeweils ein Rückhalteelement angeordnet.

Die U.S.-Patentanmeldung US 2009/0259107 A1 schlägt ein chirurgisches Retraktionssystem vor, bei dem zwei einteilige Spreizarme in einem in einer Retraktionsrichtung veränderlichem Abstand zueinander gehalten sind. Die Spreizarme weisen jeweils drei Rückhalteelemente auf.

Aus der US 2007/0161865 A1 ist eine chirurgische Retraktionsvorrichtung bekannt, bei der diesem Problem partiell Rechnung getragen wird, indem die Haltevorrichtung leicht bogenförmig ausgebildet wird, so dass die an den freien Enden der Spreizarme angeordneten Rückhalteelemente beim Retrahieren eine leicht bogenförmige Bewegung ausführen. Eine weitere Anpassung ermöglicht ein Spreizarm, der in der Spreizebene verschwenkbar an der Haltevorrichtung gehalten ist. Letzteres verlangt jedoch ein zusätzliches Eingreifen und Justieren und macht den Vorgang komplizierter und zeitaufwändiger.

Aufgabe der vorliegenden Erfindung ist es, die eingangs genannte Retraktionsvorrichtung für die Sternotomie so weiterzubilden, dass eine schonendere Eröffnung des geteilten Sternums möglich wird.

Diese Aufgabe wird erfindungsgemäß durch eine chirurgische Retraktionsvorrichtung mit den Merkmalen des Anspruchs 1 gelöst.

Aufgrund der positionsveränderlichen Lagerung in Retraktionsrichtung von mindestens einem der Rückhalteelemente gegenüber einem Spreizarm ist es möglich, bei der Eröffnung des Sternums durch die Retraktionsvorrichtung die Kräfte, die auf die cranial und caudal angeordneten Rippenbögen wirken, in einem gewissen Umfang zu verändern und anzupassen. Dies gelingt bei vielen der Ausführungsformen der Erfindung, ohne dass der Chirurg hierzu eingreifen oder nachjustieren muss.

Die an das Sternum anschließenden Rippen werden dadurch gleichmäßiger belastet, so dass die Gefahr von Spannungsspitzen an einzelnen Rippen, welche schlimmstenfalls zu Brüchen führen können, erheblich zurückgedrängt werden kann.

Ist ein Paar an positionsveränderlich gehaltenen Rückhalteelementen vorhanden, kann dieses an einem gemeinsamen Spreizarm gehalten sein oder bevorzugt in gegenüber liegenden Positionen an jeweils einem der Spreizarme, beispielsweise jeweils benachbart zum an der Haltevorrichtung gehaltenen Ende der Spreizarme.

Am meisten bevorzugt ist es, wenn alle Rückhalteelemente positionsveränderlich an den Spreizarmen gehalten sind.

Für die positionsveränderliche Lagerung gibt es mehrere bevorzugte Möglichkeiten, die im Folgenden diskutiert werden sollen. Sind mehrere der Rückhalteelemente einer erfindungsgemäßen Retraktionsvorrichtung positionsveränderlich gehalten, können sie in unterschiedlicher Weise entsprechend den verschiedenen noch zu diskutierenden Möglichkeiten an den Spreizarmen gehalten sein.

Gemäß einer ersten bevorzugten Ausführungsform der Erfindung ist das mindestens eine Rückhalteelement in seiner Position in Retraktionsrichtung gegenüber dem Spreizarm federelastisch gelagert. Durch die federelastische Lagerung gelingt die Adaption der auf das geteilte Sternum wirkenden Kräfte auch bei der (nicht erfindungsgemäßen) Verwendung einstückiger, starrer Spreizarme mit einem merklichen Effekt.

Weiter bevorzugt ist ein Paar der Rückhalteelemente federelastisch an dem oder den Spreizarmen gelagert, am meisten bevorzugt sind alle Rückhalteelemente in Retraktionsrichtung federelastisch an den Spreizarmen gelagert. Erfindungsgemäß sind bei der chirurgischen Retraktionsvorrichtung beide Spreizarme in zwei Abschnitte unterteilt, wobei ein erster Abschnitt des Spreizarms im Wesentlichen starr an der Haltevorrichtung gehalten und ein zweiter Abschnitt dieses Spreizarms am ersten Abschnitt um eine Achse senkrecht zur Spreizebene schwenkbar gehalten ist. Hier wir die Positionsveränderung durch ein Verschwenken des zweiten Teils des Spreizarms während des Retraktionsvorgangs wiederum ohne manuelles Eingreifen des Chirurgen erzielt.

Bevorzugt wird bei einer zweiten Ausführungsform der zweite Abschnitt in einer Ausgangsstellung federelastisch im Wesentlichen kollinear oder koaxial mit dem ersten Abschnitt gehalten. Bei Krafteinwirkung parallel zur Spreizebene während der Retraktion wird dann der zweite Abschnitt gegen den ersten Abschnitt gegen die Federelastizität verschwenkt.

Weiter bevorzugt wird die Verschwenkbewegung mittels eines Anschlags auf einen vorgegebenen Winkel begrenzt.

Bei der erfindungsgemäßen Retraktionsvorrichtung sind beide Spreizarme in zwei Abschnitte unterteilt, wobei ein erster Abschnitt jedes Spreizarms im Wesentlichen starr an der Haltevorrichtung gehalten ist und der zweite Abschnitt der Spreizarme am ersten Abschnitt um eine Schwenkachse senkrecht zur Spreizebene schwenkbar gehalten ist.

Bei einer nicht erfindungsgemäßen Ausführungsform sind ein erstes Rückhalteelement am ersten, starr mit der Haltevorrichtung verbundenen Abschnitt des Spreizarms und ein zweites Rückhalteelement am zweiten, verschwenkbaren Abschnitt des Spreizarms gehalten.

Bei einer Variante dieser Ausführungsform kann vorgesehen sein, dass der zweite Abschnitt bei Bedarf in einer gegenüber dem ersten Abschnitt verschwenkten Position arretierbar ist.

Bei einer weiteren Variante dieser Ausführungsform ist vorgesehen, dass der zweite Abschnitt mittels eines Antriebs gegenüber dem ersten Abschnitt verschwenkbar ist, wobei der Antrieb vorzugsweise selbsthemmend ausgebildet ist.

Vorzugsweise weist bei der zweiten Ausführungsform der erfindungsgemäßen Retraktionsvorrichtung der erste Abschnitt des oder der Spreizarme eine Länge auf, die größer ist als die Hälfte der gesamten Längsausdehnung des Spreizarms.

Bei der erfindungsgemäßen Retraktionsvorrichtung sind an dem zweiten Abschnitt zwei Rückhalteelemente angeordnet, wobei die Schwenkachse, die den ersten mit dem zweiten Abschnitt verbindet, vorzugsweise zwischen den Rückhalteelementen angeordnet ist. Der zweite Abschnitt überlappt bei einer dritten Ausführungsform in seiner Längsausdehnung vorzugsweise ungefähr zur Hälfte mit der Längsausdehnung des ersten Abschnitts des Spreizarms. Am ersten Abschnitt des Spreizarms ist dann vorzugsweise kein Rückhalteelement angeordnet.

Bei der zweiten und dritten Ausführungsform ist der erste Abschnitt des Spreizarms gemäß einer Variante im Wesentlichen gerade ausgebildet. Bei einer weiteren Variante ist das freie Ende des ersten Abschnitts, an dem der zweite Abschnitt schwenkbar gelagert ist, abgewinkelt ausgebildet, wobei der abgewinkelte Bereich in der Spreizebene gegen den jeweils anderen Spreizarm vorspringt. Der zweite Abschnitt ist dann an dem freien Ende des abgewinkelten Teils in der Spreizebene schwenkbar gelagert.

Auch hier gibt es mehrere Varianten der Lagerung des zweiten Abschnitts am ersten Abschnitt des Spreizarms.

Gemäß einer ersten Variante ist der zweite Abschnitt des Spreizarms federelastisch in einer Ausgangsstellung mit vorzugsweise paralleler Ausrichtung zum ersten Abschnitt gehalten.

Gemäß einer zweiten Variante ist der zweite Abschnitt in einer vorgegebenen Schwenkstellung an dem ersten Abschnitt arretierbar.

Gemäß einer dritten Variante ist die Verschwenkbarkeit des zweiten Abschnitts gegenüber dem ersten Abschnitt mittels eines Anschlags begrenzt.

Gemäß einer vierten Variante ist der zweite Abschnitt gegenüber dem ersten Abschnitt mittels eines Antriebs gegenüber dem ersten Abschnitt verschwenkbar gelagert, wobei der Antrieb vorzugsweise selbsthemmend ausgebildet ist.

Generell ist bei der zweiten und dritten Ausführungsform bevorzugt, wenn die Rückhalteelemente in einem im Wesentlichen gleichen Abstand zur Schwenkachse des zweiten Abschnitts des Spreizarms angeordnet sind. Die Schwenkachse ist dabei vorzugsweise im Wesentlichen mittig zur Längsrichtung des zweiten Abschnitts angeordnet.

Des Weiteren kann die Schwenkachse des zweiten Abschnitts an mehreren Positionen entlang der Längsrichtung des zweiten Abschnitts festlegbar sein. Bevorzugt weist der zweite Abschnitt hierfür mehrere Lagerbuchsen auf.

Bei einer weiteren Alternative ist vorgesehen, dass die Schwenkachse von einem Lagerelement gebildet wird, welches verschieblich an dem ersten Abschnitt gehalten ist.

Gemäß einer vierten Ausführungsform der Erfindung ist der zweite Abschnitt an einem biegeelastischen Bereich des ersten Abschnitts gehalten.

Ist der erste Abschnitt eines Spreizarms an seinem freien Ende abgewinkelt ausgebildet, kann bei einer nicht erfindungsgemäßen Variante der abgewinkelte Bereich in der Spreizebene gabelförmig geteilt sein. Dann wird der zweite Abschnitt vorzugsweise in zwei separate Längselemente unterteilt, von denen jedes mit einem der Gabelzinken verbunden ist.

Auch der zweite Spreizarm selbst kann in der Spreizebene biegeelastisch ausgebildet werden und so weiter zur positionsveränderlichen Halterung der Rückhalteelemente führen oder beitragen.

Die Erfindung betrifft des Weiteren eine chirurgische Retraktionsvorrichtung der eingangs beschriebenen Art, bei der die beiden Spreizarme in Längsrichtung gesehen in zwei Segmente unterteilt sind, wobei ein erstes Segment mit einem Ende an der Haltevorrichtung bezüglich der Spreizebene in einer im Wesentlichen unveränderlichen Ausrichtung gehalten ist und wobei ein zweites Segment am freien Ende des ersten Segments um eine Schwenkachse in einer Ebene senkrecht zur Spreizebene verschwenkbar gehalten ist. Typischerweise verläuft die Schwenkachse quer zur Längsrichtung der Spreizarme. Vorzugsweise ist der Schwenkwinkel des zweiten Segments mittels eines Anschlags begrenzt, so dass vorzugsweise eine Schwenkbewegung des zweiten Segments nur in einer Richtung aus der Spreizebene heraus möglich ist. Gemäß einem anderen bevorzugten Aspekt ist die Länge des ersten Segments kleiner als die des zweiten Segments und beträgt vorzugsweise ca. 30 % der Gesamtlänge des Spreizarms oder weniger.

Weiter bevorzugt sind die Rückhalteelemente ausschließlich an dem zweiten Segment der Spreizarme direkt oder indirekt gehalten.

Die Unterteilung der Spreizarme in zwei Segmente, wobei das zweite Segment am ersten schwenkbar gehalten ist, hat den Vorteil, dass sich die Geometrie der chirurgischen Retraktionsvorrichtung an die Form des Rumpfes des Patienten anpassen kann, so dass sich der Platzbedarf der Vorrichtung und damit gleichzeitig eine mögliche Behinderung des Chirurgen vermindern lässt.

Soweit die Spreizarme in einen ersten und einen zweiten Abschnitt unterteilt sind, ist die Gelenkverbindung zwischen erstem und zweitem Segment vorzugsweise im ersten Abschnitt angeordnet.

Es sei an dieser Stelle betont, dass die Unterteilung der Spreizarme in einen ersten und einen zweiten Abschnitt einerseits und in ein erstes und ein zweites Segment andererseits keine konkurrierenden Konzepte darstellen, sondern zwei Konzepte, die gleichzeitig bei einer Ausführungsform einer erfindungsgemäßen chirurgischen Retraktionsvorrichtung realisiert sein können.

Bei all den zuvor beschriebenen Ausführungsformen mit all ihren Varianten gilt, dass die Rückhalteelemente zusätzlich zu der dort ggf. gegebenen Positionsveränderlichkeit federelastisch an den Spreizarmen bzw. deren ersten und/oder zweiten Abschnitten gehalten sein können. Hier lässt sich die federelastische Lagerung zu einer weiteren Verbesserung der Adaption der auf das geteilte Sternum wirkenden Kräfte einsetzen.

Erfindungsgemäß sind die Rückhalteelemente, vorzugsweise in ihrem Abstand, entlang des Spreizarms verstellbar. Dies ermöglicht eine weitere Optimierung der erfindungsgemäßen Retraktionsvorrichtung bezüglich der Einleitung der Kräfte auf das an den Rückhalteelementen anliegende Knochenmaterial.

Um eine möglichst günstige Geometrie für das Einführen der Retraktionsvorrichtung in den Spalt des geteilten Sternums zu erzielen, werden die Rückhalteelemente der beiden Spreizarme in deren Längsrichtung zueinander versetzt angeordnet. Die Spreizarme können so anfänglich einander maximal angenähert werden.

Weiter bevorzugte Rückhalteelemente, die bei den erfindungsgemäßen Retraktionsvorrichtungen zum Einsatz kommen können, sind zweiteilig ausgebildet.

Hierbei wird bevorzugt ein erstes Teil des Rückhalteelements als Lagerteil am Spreizarm gehalten und das zweite Teil, das als Rückhaltefläche ausgebildet ist, beweglich, insbesondere um eine zur Spreizebene senkrecht und/oder parallel ausgerichtete Achse schwenkbar, an dem ersten Teil gelagert.

Bevorzugte Rückhalteelemente weisen eine Rückhaltefläche mit einer maximalen Ausdehnung parallel zur Längsrichtung des Spreizarms in einem Bereich auf, in dem typischerweise das Knochenmaterial zur Anlage kommt, während deren Ausdehnung benachbart zum Spreizarm einerseits und/oder am freien Ende der Rückhaltefläche andererseits geringer gewählt wird. Dies schafft dem Chirurgen eine bessere Sicht, beispielsweise beim Präparieren einer Arteria Mammaria.

Insbesondere vorteilhaft ist ein stegartig schmal ausgebildetes freies Ende der Rückhaltefläche, das zudem abgekröpft ausgebildet sein kann, so dass das Knochenmaterial des Sternums sicher erfasst wird.

Bevorzugte Rückhalteelemente weisen ein Lagerteil auf, bei dem eine Rastvorrichtung integriert ist, mit der mit dem Spreizarm eine lösbare Rastverbindung herstellbar ist. Dazu weist der Spreizarm bevorzugt vorgegebene Rastpositionen auf, so dass ein definiertes Platzieren der Rückhalteelemente entlang der Spreizarme möglich ist. So können insbesondere einander an verschiedenen Spreizarmen gegenüber liegend angeordnete Rückhalteelemente genau aufeinander ausgerichtet platziert werden.

Diese und weitere Vorteile der vorliegenden Erfindung werden im Folgenden anhand der Zeichnung noch näher erläutert. Es zeigen im Einzelnen:
- Figur 1:: eine Ausführungsform einer nicht erfindungsgemäßen Retraktionsvorrichtung;
- Figur 2:: eine weitere Ausführungsform einer nicht erfindungsgemäßen Retraktionsvorrichtung;
- Figur 3:: eine Ausführungsform einer erfindungsgemäßen Retraktionsvorrichtung;
- Figuren 4A und 4B:: eine Variante der Ausführungsform der Retraktionsvorrichtung der Figur 3;
- Figur 5:: eine weitere Variante der Ausführungsform der Retraktionsvorrichtung der Figur 3;
- Figur 6:: eine weitere Ausführungsform einer nicht erfindungsgemäßen Retraktionsvorrichtung;
- Figur 7:: eine weitere Ausführungsform einer nicht erfindungsgemäßen Retraktionsvorrichtung;
- Figur 8:: eine weitere Ausführungsform einer erfindungsgemäßen Retraktionsvorrichtung; und
- Figuren 9A und 9B:: ein bevorzugtes Rückhalteelement für eine erfindungsgemäße Retraktionsvorrichtung.

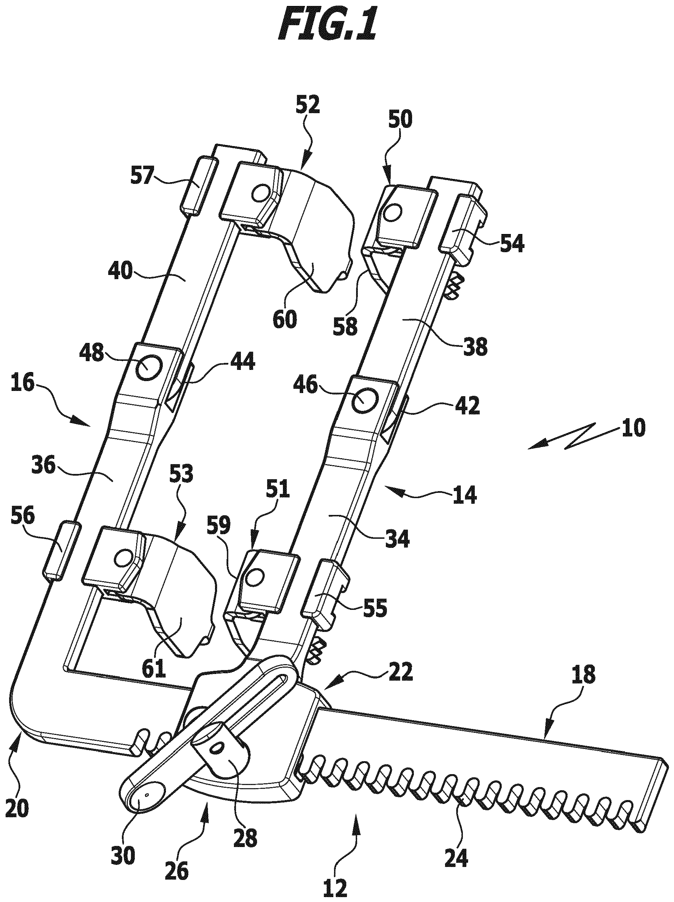

Figur 1 zeigt eine Ausführungsform einer nicht erfindungsgemäßen Retraktionsvorrichtung 10 mit einer Haltevorrichtung 12 sowie zwei an der Haltevorrichtung 12 angeordnete, in variablem Abstand zueinander positionierbare Spreizarme 14 und 16. Die beiden Spreizarme 14 und 16 sind im Wesentlichen rechtwinklig zur Längsrichtung der Haltevorrichtung 12 und parallel zueinander angeordnet und definieren die Spreizebene der Retraktionsvorrichtung 10.

Die Haltevorrichtung 12 umfasst eine im Wesentlichen gerade Schiene 18, an deren einem Ende 20 ein Ende des zweiten Spreizarms 16 einstückig angeformt ist. Der Spreizarm 14 ist in Längsrichtung der Schiene 18 verschieblich angeordnet. Der Spreizarm 14 weist hierzu ein Lagerelement 22 auf, welches die Schiene 18 der Haltevorrichtung 12 umgreift.

Die Schiene 18 weist einen im Wesentlichen rechteckigen Querschnitt auf, bei dem an einer Schmalseite eine Zahnreihe 24 ausgebildet ist. Das Lagerelement 22 weist einen Antriebsmechanismus 26 mit einem mit der Zahnreihe 24 kämmenden Drehkörper auf (im Einzelnen nicht gezeigt), beispielsweise einem Zapfenrad oder einem Zahnrad. Dieser Drehkörper ist auf einer Lagerwelle 28 drehfest gehalten, welche sich senkrecht zur Längsachse der Schiene 18 und zur Spreizebene erstreckt und drehbar in dem Lagerelement 22 gehalten ist. An ihrem freien, in der Figur nach oben weisenden Ende trägt die Lagerwelle 28 ein Griffelement 30, mit dem der Drehkörper rotiert werden kann und in der Folge der Abstand der beiden Spreizarme 14 und 16 zueinander entlang der Schiene 18 verändert werden kann.

Die Spreizarme 14, 16 weisen jeweils gerade erste Abschnitte 34, 36 auf, die benachbart zur Haltevorrichtung 12 angeordnet sind, sowie gerade zweite Abschnitte 38, 40, die verschwenkbar am freien Ende der ersten Abschnitte 34, 36 der Spreizarme 14 bzw. 16 gehalten sind. Die Länge der ersten Abschnitte 34, 36 ist etwas größer als die Länge der zweiten Abschnitte 38, 40.

Zur Halterung der zweiten Abschnitte 38, 40 weisen die ersten Abschnitte 34, 36 der Spreizarme 14 bzw. 16 gegabelt ausgebildete Lagerelemente 42, 44 auf, in denen jeweils ein haltevorrichtungsseitiges Ende der zweiten Abschnitte 38, 40 mittels eines Zapfenlagers 46 bzw. 48 in der Spreizebene schwenkbar gehalten ist.

An den Spreizarmen 14, 16 sind jeweils zwei Rückhalteelemente 50, 51 bzw. 52, 53 gehalten. Je eines der Rückhalteelemente 51 bzw. 53 ist an einem ersten Abschnitt 34 bzw. 36 und jeweils eines der Rückhalteelemente 50 bzw. 52 an einem zweiten Abschnitt 38 bzw. 40 der Spreizarme 14 und 16 angeordnet. Die Rückhalteelemente 50, 51, 52, 53 sind jeweils zweiteilig ausgebildet mit einem Lagerteil 54, 55, 56, 57 sowie daran um eine Achse senkrecht zur Spreizebene drehbar gehaltene Rückhalteflächen 58, 59, 60, 61. Die Lagerteile 54, 55, 56, 57 sind bevorzugt so ausgebildet, dass sie verschieblich an ihren jeweils zugeordneten Abschnitten der Spreizarme 14 bzw. 16 gehalten und in unterschiedlichen Positionen fixierbar sind. Aufgrund der etwas größeren Länge der ersten Abschnitte 34, 36 gegenüber der Länge der zweiten Abschnitte 38, 40 ist sichergestellt, dass die Rückhalteelemente symmetrisch zu den Zapfenlagern 46, 48, unter Ausnutzung der gesamten Länge der zweiten Abschnitte 38, 40, positioniert werden können.

Aufgrund der Aufteilung der Spreizarme 14 und 16 in jeweils zwei Abschnitte 34, 38 sowie 36, 40 und der verschwenkbaren Lagerung der zweiten Abschnitte an den ersten Abschnitten ist eine positionsveränderliche Lagerung der Rückhalteelemente 58, 59, 60, 61 in der Spreizebene geschaffen. Beim Spreizen des längs geteilten Sternums eines Patienten können die dabei aufzuwendenden erheblichen Kräfte über die Länge des geteilten Sternums gesehen unterschiedlich dosiert eingeleitet werden, so dass eine schonendere Eröffnung des geteilten Sternums möglich ist.

Die verschwenkbare Lagerung der zweiten Abschnitte 38, 40 an den ersten Abschnitten 34, 36 der Spreizarme 14, 16 lässt sich auf verschiedene Weisen realisieren.

Zunächst können die zweiten Abschnitte 38, 40 an den ersten Abschnitten 34, 36 federelastisch in einer geraden Ausrichtung gehalten werden, wie in Figur 1 gezeigt. Dies kann z.B. mittels Zug-, Druck- oder Torsionsfedern geschehen, wobei die Federraten für die einzelnen zweiten Abschnitte verschieden und insbesondere auch einstellbar sein können. Erst beim Auftreten der Retraktionskräfte aufgrund der Betätigung des Antriebsmechanismus' 26 des Lagerelements 22 verschwenken die zweiten Abschnitte 38, 40 in der Spreizebene gegen die Federkraft der Lagerung. Die Winkel, um den die zweiten Abschnitte aus der geraden Konfiguration ausgelenkt werden können, werden bevorzugt durch einen Anschlag begrenzt. Bevorzugt begrenzen die Anschläge die Auslenkung auf jeweils ca. 30°.

Des Weiteren können die Zapfenlager 46, 48 durch Schwenkantriebe ersetzt werden, mit denen ein Auslenk-Winkel vorgegeben werden kann.

Auch kann eine Vorrichtung zum Arretieren der zweiten Abschnitte in einer einmal erzielten Verschwenkstellung vorgesehen sein.

Die Länge der ersten Abschnitte 34, 36 ist größer als die Hälfte der gesamten Längsausdehnung der Spreizarme 14 bzw. 16, so dass die Schwenklager 46, 48 ungefähr in der Mitte der Längsausdehnung der Spreizarme 14, 16 angeordnet sind.

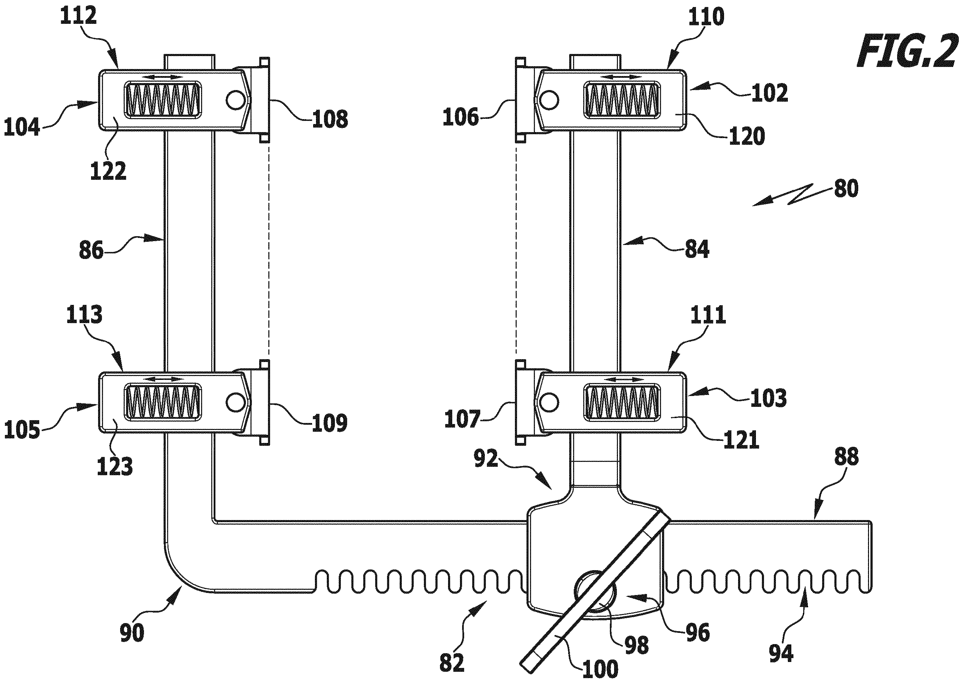

Eine weitere Ausführungsform einer Retraktionsvorrichtung 80 ist in der Figur 2 gezeigt. Die Retraktionsvorrichtung 80 umfasst eine Haltevorrichtung 82 sowie zwei parallel angeordnete, gerade, starre Spreizarme 84, 86, wovon der erste Spreizarm 84 einseitig und längsverschieblich an der Halterung 82 bzw. deren Schiene 88 gehalten wird, während der zweite Spreizarm 86 an einem Ende 90 der Haltevorrichtung 82 einstückig im rechten Winkel angeformt ist. Die beiden Spreizarme 84, 86 definieren die Spreizebene der Retraktionsvorrichtung 80.

Die Schiene 88 ist über den größten Teil ihrer Längsausdehnung an einer Schmalseite ihres rechteckigen Querschnitts mit einer Zahnreihe 94 versehen.

Der Spreizarm 84 ist mit einem Lagerelement 92 verschieblich an der Schiene 88 gehalten. Das Lagerelement 92 beinhaltet einen Antriebsmechanismus 96, der einen Drehkörper umfasst (im Einzelnen nicht dargestellt), der beispielsweise ein Zahnrad umfasst, welches in die Zahnreihe 84 eingreift. Der Antriebsmechanismus 96 umfasst eine in dem Lagerelement 92 gehaltene Welle 98, die mittels eines Griffelements 100 rotiert werden kann, so dass sich daraus dann eine lineare Verschiebung des Spreizarms 84 entlang der Längsrichtung der Haltevorrichtung 82 bzw. ihrer Schiene 88 ergibt und somit eine Veränderung des Abstandes der beiden Spreizarme 84 und 86.

An den beiden starren Spreizarmen 84, 86 sind paarweise Rückhalteelemente 102, 103 sowie 104, 105 angeordnet. Die Rückhalteelemente 102, 103, 104, 105 sind vorzugsweise entlang der Längsrichtung der Spreizarme 84 bzw. 86 verschieblich an diesen gelagert, wobei sie weiter vorzugsweise an jeweils vorgegebenen Positionen fixierbar sind.

Weiter bevorzugt entspricht die Anzahl der vorhandenen Rückhalteelemente der Zahl der am Sternum gehaltenen Rippen.

Die Rückhalteelemente 102, 103, 104, 105 sind wiederum mehrteilig ausgebildet und umfassen ein Halteteil 110, 111, 112, 113, an dem Rückhalteflächen 106, 107, 108, 109, vorzugsweise um eine Achse senkrecht zur Spreizebene, verschwenkbar gehalten sind. Die Halteteile 110, 111, 112, 113 umfassen ein Lagerteil (im Einzelnen nicht dargestellt), welches den im Wesentlichen rechteckigen Querschnitt der Spreizarme 84 bzw. 86 umgreift, und eine daran federelastisch vorgespannte Halterung 120, 121, 122, 123. Die federelastische Vorspannung der Halterungen 120, 121, 122, 123 ist vorzugsweise für die einzelnen Rückhalteelemente 106, 107, 108, 109 ihrer Position angepasst gewählt, so dass jeder Bereich des Sternums entsprechend den anatomischen Gegebenheiten beim Retrahieren mit Kraft beaufschlagt werden kann. Aufgrund der federelastischen Halterung der Rückhalteelemente 106, 107, 108, 109 ist wiederum die Möglichkeit der positionsveränderlichen Lagerung der Rückhalteelemente bzw. der mit den Knochenmaterialien in Kontakt kommenden entsprechenden Rückhalteflächen gegeben, so dass die erheblichen Kräfte, die beim Spreizen eines Sternums aufzubringen sind, wieder dosiert und der unterschiedlichen Elastizität des Sternums und der daran angrenzenden Rippenbögen angepasst ausgeübt werden können.

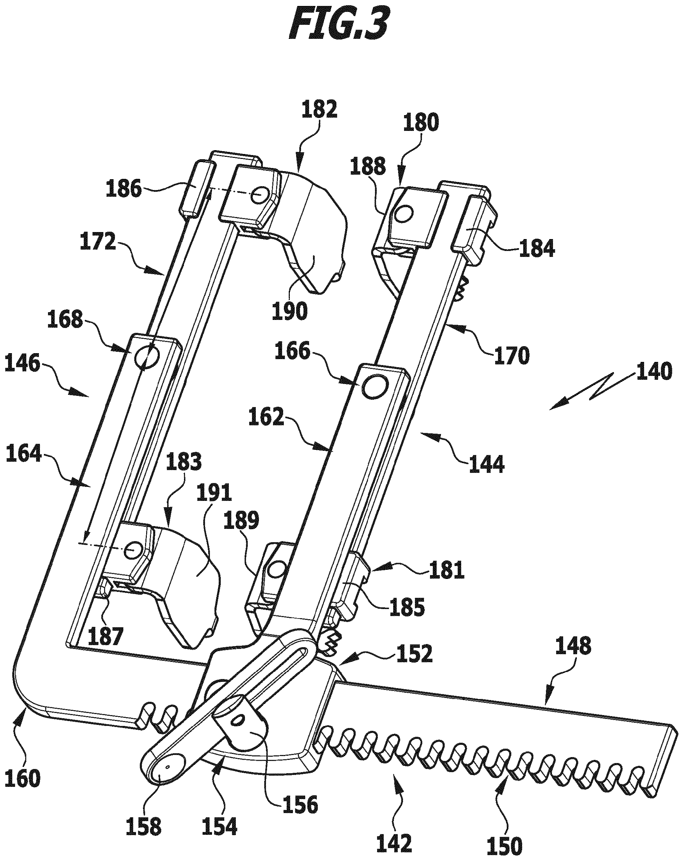

Figur 3 zeigt eine Ausführungsform einer erfindungsgemäßen Rückhaltevorrichtung 140 mit einer Haltevorrichtung 142 sowie zwei einseitig an der Haltevorrichtung 142 gehaltenen Spreizarmen 144, 146. Die Haltevorrichtung 142 umfasst eine im Wesentlichen gerade ausgebildete Schiene 148 mit einem im Wesentlichen rechteckigen Querschnitt. An einer ihrer Schmalseiten weist die Schiene 148 eine Zahnreihe 150 auf.

Der Spreizarm 144 ist entlang der Längsrichtung der Schiene 148 verfahrbar, so dass der Spreizarm 144 dem Spreizarm 146 angenähert oder der Abstand der beiden Spreizarme voneinander vergrößert werden kann. Zu diesem Zweck ist der Spreizarm 144 mittels eines Lagerelements 152 an der Haltevorrichtung 142 bzw. dessen Schiene 148 verschieblich gehalten. Das Lagerelement 152 umfasst einen Antriebsmechanismus 154, der einen Drehkörper (im Einzelnen nicht dargestellt), beispielsweise in Form eines Zapfenrades oder eines Zahnrades, umfasst, welcher auf einer Antriebswelle 156 drehfest montiert ist, welche nach oben aus dem Lagerelement 152 heraussteht und dort mit einem Griffelement 158 bestückt ist.

Durch Rotieren der Welle 156 mit Hilfe des Griffelements 158 kann über die Rotationsbewegung des Drehkörpers eine Verschiebung des Spreizarms 144 entlang der Schiene 148 erfolgen. Vorzugsweise ist der Drehkörper der Antriebsvorrichtung 154 so ausgestaltet, dass sich eine selbsthemmende Verbindung zwischen dem Drehkörper und der Zahnreihe 150 der Schiene 148 ergibt.

Der zweite Spreizarm 146 ist mit einem Ende an ein Ende 160 der Haltevorrichtung 142 einstückig angeformt. Die beiden Spreizarme 144, 146 liegen in einer Ebene, der Spreizebene.

Die beiden Spreizarme 144, 146 sind zweiteilig ausgebildet, wobei ein gerader erster Abschnitt 162, 164 an der Haltevorrichtung 142 in einem rechten Winkel gehalten ist. An ihrem freien, der Haltevorrichtung 142 entgegen gesetzten Ende tragen die beiden ersten Abschnitte 162, 164 Lagerstellen 166, 168, an denen jeweils ein gerader zweiter Abschnitt 170, 172 der Spreizarme 144, 146 verschwenkbar gehalten ist. Bevorzugt können die zweiten Abschnitte in einer parallelen Ausrichtung zu den ersten Abschnitten arretiert werden, so dass ein Applizieren der Retraktionsvorrichtung vereinfacht ist.

In einer abgewandelten, nicht erfindungsgemäßen, Ausführungsform ist nur ein Spreizarm zweiteilig ausgebildet, wobei dann ein größerer Verschwenkwinkel des zweiten Abschnitts des geteilten Spreizarms zuzulassen ist. Beim Retraktionsvorgang verändert dann die Haltevorrichtung ihre Position. Diese Ausführungsform hat den Vorteil, dass die Zahl der benötigten Teile verringert ist.

Anders als bei der Ausführungsform der Figur 1 sind die zweiten Abschnitte 170, 172 nicht an einem ihrer Enden mit dem ersten Abschnitt 162, 164 verbunden, sondern mittig drehbar um eine Achse senkrecht zur Spreizebene an dem freien Ende der ersten Abschnitte 162, 164 der Spreizarme 144, 146 gehalten. In der Folge überlappt der zweite Abschnitt 170, 172 ungefähr zur Hälfte seiner Länge mit dem freien Ende der ersten Abschnitte 162, 164 der Spreizarme 144, 146.

Die Retraktionsvorrichtung 140 weist ebenfalls vier Rückhalteelemente 180, 181, 182, 183 auf, die allerdings paarweise an den zweiten Abschnitten 170, 172 gehalten sind. Vorzugsweise sind die Rückhalteelemente 180, 181, 182, 183 längsverschieblich an den zweiten Abschnitten 170 bzw. 172 gehalten und in ihren jeweils vorgegebenen Positionen fixierbar.

Die Rückhalteelemente 180, 181, 182, 183 sind, ähnlich wie die Rückhalteelemente der Ausführungsform der Figur 1, zweiteilig ausgebildet, wobei ein erster Teil ein Halteteil 184, 185, 186, 187 bildet, an dem Rückhalteflächen 188, 189, 190, 191 um eine Achse senkrecht zur Spreizebene verschwenkbar gehalten sind.

Beim Aufbringen von Retraktionskräften am längs geteilten Sternum richten sich die zweiten Abschnitte 170, 172 mit ihren Rückhalteelementen 180, 181, 182, 183 entsprechend den von dem Knochenmaterial des Sternums und den daran anschließenden Rippenbögen ausgeübten Widerstandskräften automatisch aus, so dass wiederum eine positionsveränderliche Lagerung der Rückhalteelemente gegeben ist und auch hier eine dosierte Krafteinleitung entsprechend der unterschiedlichen Elastizität der einzelnen Abschnitte des geteilten Sternums mit den daran anschließenden Rippenbögen möglich ist. Diese Ausführungsform der Erfindung ermöglicht die Anpassung der Kräfte ohne Federelemente, was Vorteile bei den nicht linearen Federraten der Rippen in der Sternotomie ergibt. Insbesondere reagiert auch diese erfindungsgemäße Retraktionsvorrichtung selbsttätig individuell auf unterschiedliche Brustkörbe der Patienten entsprechend dem festgelegten Hebel-/Kräfteverhältnis.

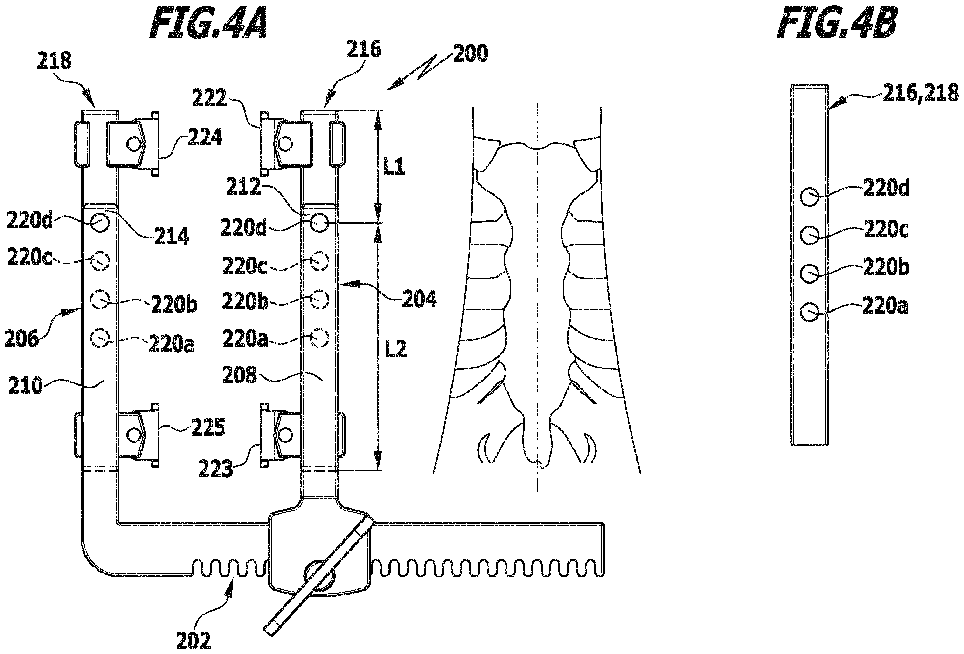

Die Figuren 4A und 4B zeigen eine Variante der erfindungsgemäßen Retraktionsvorrichtung 140 der Figur 3 in Form einer Retraktionsvorrichtung 200. Die Retraktionsvorrichtung 200 umfasst eine Haltevorrichtung 202 sowie zwei daran einseitig und in einer Ebene, der Spreizebene, gehaltene Spreizarme 204, 206.

Die Spreizarme 204, 206 sind wiederum zweiteilig ausgebildet und mit einem geraden ersten Abschnitt 208, 210 jeweils einseitig an der Haltevorrichtung 202 gehalten. Sie tragen an ihrem freien, der Haltevorrichtung 202 entgegen gesetzten Ende 212, 214 einen geraden zweiten, um eine Achse senkrecht zur Spreizebene schwenkbaren Abschnitt 216, 218. Die zweiten Abschnitte 216, 218 überlappen in großen Teilen wiederum mit dem zugeordneten ersten Abschnitt 208, 210 der Spreizarme 204, 206, so dass die beiden Paare an Rückhalteelementen 222, 223, 224, 225 an den jeweils zweiten Abschnitten 216, 218 der Spreizarme 204, 206 gehalten sind.

Während jedoch bei der Ausführungsform der Figur 3 die zweiten Abschnitte mittig an dem zugehörigen ersten Abschnitt unveränderlich schwenkbar festgelegt sind, ist bei der Ausführungsform der Figuren 4A und 4B vorgesehen, dass die Position der Lagerung am zweiten Abschnitt 216, 218 verändert werden kann, und vorzugsweise sind hierzu an dem zweiten Abschnitt 216, 218 bereits mehrere Lagerstellen 220a, 220b, 220c und 220d ausgebildet.

Somit lassen sich die Hebelverhältnisse der Abschnitte L1 und L2 der zweiten Abschnitte 216, 218 der Spreizarme 204, 206 verändern und so entsprechend der Gegebenheiten bei einem Patienten anpassen, wie dies schematisch in Figur 4 gezeigt ist.

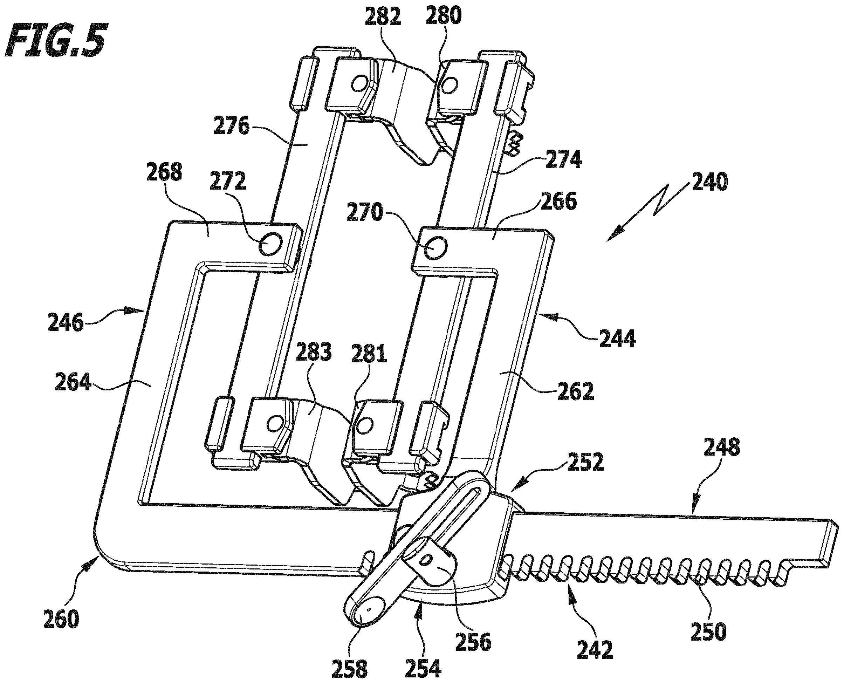

Figur 5 zeigt eine weitere Ausführungsform der erfindungsgemäßen Rückhaltevorrichtung 240 mit einer Haltevorrichtung 242 sowie zwei einseitig an der Haltevorrichtung 242 gehaltenen Spreizarmen 244, 246. Die Haltevorrichtung 242 umfasst eine im Wesentlichen gerade ausgebildete Schiene 248 mit einem im Wesentlichen rechteckigen Querschnitt. An einer ihrer Schmalseiten weist die Schiene 248 eine Zahnreihe 250 auf.

Der Spreizarm 244 ist entlang der Längsrichtung der Schiene 248 verfahrbar, so dass der Spreizarm 244 dem Spreizarm 246 angenähert oder der Abstand der beiden Spreizarme voneinander vergrößert werden kann. Zu diesem Zweck ist der Spreizarm 244 mittels eines Lagerelements 252 an der Haltevorrichtung 242 bzw. deren Schiene 248 verschieblich gehalten. Das Lageelement 252 umfasst einen Antriebsmechanismus 254, der einen Drehkörper (im Einzelnen nicht dargestellt), beispielsweise in Form eines Zapfenrades oder eines Zahnrades, umfasst, welcher auf einer Antriebswelle 256 drehfest montiert ist, welche nach oben aus dem Lagerelement 252 heraussteht und dort mit einem Griffteil 258 bestückt ist.

Durch Drehen des Griffteils 258 und damit der Antriebswelle 256 kann über die Rotationsbewegung des Drehkörpers eine Verschiebung des Spreizarms 244 entlang der Schiene 248 erfolgen. Vorzugsweise ist der Drehkörper des Antriebsmechanismus' 254 so gestaltet, dass sich eine selbsthemmende Verbindung zwischen dem Drehkörper und der Zahnreihe 250 der Schiene 248 ergibt.

Der zweite Spreizarm 246 ist mit einem Ende an ein Ende 260 der Haltevorrichtung 242 einstückig angeformt. Die beiden Spreizarme 244, 246 liegen in einer Ebene, der Spreizebene.

Die beiden Spreizarme 244, 246 sind zweiteilig ausgebildet, wobei ein erster Abschnitt 262 bzw. 264 an der Haltevorrichtung 142 in einem rechten Winkel gehalten ist. An ihrem freien, der Haltevorrichtung 242 entgegengesetzten Ende weisen die beiden ersten Abschnitte 262, 264 gegeneinander vorspringende abgewinkelte Bereiche 266, 268 auf, an welchen Lagerstellen 270, 272 ausgebildet sind, an denen jeweils ein gerader zweiter Abschnitt 274, 276 verschwenkbar gehalten ist.

Die Retraktionsvorrichtung 240 weist vier Rückhalteelemente 280, 281, 282, 283 auf, die paarweise an den zweiten Abschnitten 274, 276, vorzugsweise verschieblich, gehalten sind.

Die Rückhalteelemente 280, 281, 282, 283 sind ähnlich wie die Rückhalteelemente 50, 51, 52, 53 der Figur 1 ausgebildet, so dass auf die dort gegebene Beschreibung der Rückhalteelemente verwiesen werden kann.

Beim Aufbringen von Retraktionskräften am längs geteilten Sternum richten sich die beiden zweiten Abschnitte 274, 276 entsprechend den von dem Knochenmaterial des Sternums und den daran anschließenden Rippenbögen ausgeübten Widerstandskräften aus, so dass auch bei dieser Ausführungsform eine positionsveränderliche Lagerung der Rückhalteelemente gegeben ist und eine dosierte Krafteinleitung entsprechend der unterschiedlichen Elastizität der einzelnen Abschnitte des geteilten Sternums mit den daran anschließenden Rippenbögen erfolgt.

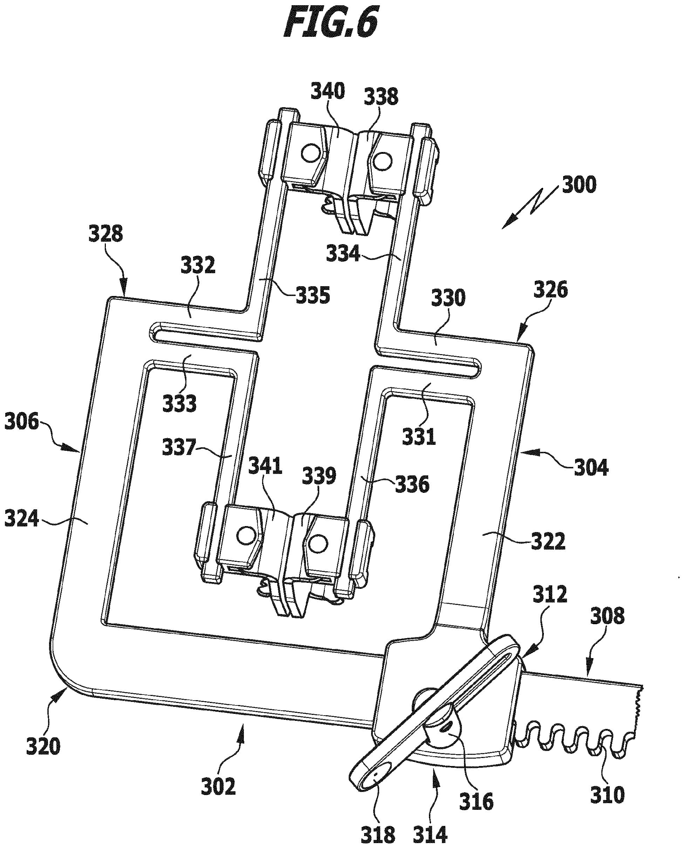

Eine weitere Ausführungsform einer Retraktionsvorrichtung soll anhand der in der Figur 6 dargestellten Retraktionsvorrichtung 300 beschrieben werden.

Die Retraktionsvorrichtung 300 weist eine Haltevorrichtung 302 sowie zwei einseitig im rechten Winkel an der Haltevorrichtung 302 gehaltene Spreizarme 304, 306 auf. Die Haltevorrichtung 302 umfasst eine im Wesentlichen gerade ausgebildete Schiene 308 mit einem im Wesentlichen rechteckigen Querschnitt, der an einer seiner Schmalseiten eine Zahnreihe 310 aufweist.

Der Spreizarm 304 ist entlang der Längsrichtung der Schiene 308 verfahrbar, so dass der Spreizarm 304 dem Spreizarm 306 angenähert oder der Abstand der beiden Spreizarme voneinander vergrößert werden kann. Zu diesem Zweck ist der Spreizarm 304 mittels eines Lagerelements 312 an der Schiene 308 verschieblich gehalten. Das Lagerelement 312 umfasst einen Antriebsmechanismus 314, der einen Drehkörper (im Einzelnen nicht dargestellt), beispielsweise in Form eines Zapfenrades oder eines Zahnrades, umfasst, welcher auf einer Antriebswelle 316 drehfest montiert ist, welche nach oben aus dem Lagerelement 312 heraussteht und dort mit einem Griffelement 318 versehen ist.

Wird die Welle 316 mit Hilfe des Griffelements 318 gedreht, kann über die Rotationsbewegung des auf der Antriebswelle 316 drehfest montierten Drehkörpers eine Verschiebung des Spreizarms 304 entlang der Schiene 308 erfolgen. Vorzugsweise ist der Drehkörper der Antriebsvorrichtung 314 so ausgestaltet, dass sich eine selbsthemmende Verbindung zwischen dem Drehkörper und der Zahnreihe 310 der Schiene 308 ergibt.

Der zweite Spreizarm 306 ist mit einem Ende an ein Ende 320 der Haltevorrichtung 302 einstückig angeformt. Die beiden Spreizarme 304, 306 liegen in einer Ebene und definieren so die Spreizebene.

Die beiden Spreizarme 304, 306 sind mehrteilig ausgebildet, wobei ein erster Abschnitt 322, 324 an der Haltevorrichtung 302 in einem rechten Winkel zur Schiene 308 gehalten ist. An ihren von der Haltevorrichtung 302 abgelegenen Enden weisen die Spreizarme 304, 306 gegeneinander weisende, in der Spreizebene liegende, abgewinkelte Bereiche 326, 328 auf, die in der Spreizebene gabelförmig geteilt sind.

Vorzugsweise bestehen die abgewinkelten Bereiche 326, 328 im Wesentlichen aus den parallel angeordneten Gabelzinken 330, 331 bzw. 332, 333.

An den freien Enden der parallelen Gabelzinken 330, 331, 332, 333 sind in etwa in einem rechten Winkeln gerade Schienenelemente 334, 335, 336, 337 angeformt, welche paarweise die Funktion eines zweiten Abschnitts der Spreizarme 304, 306 übernehmen.

Der Querschnitt der Gabelzinken 330, 331, 332, 333 und/oder der Schienenelemente 334, 335, 336, 337 wird so gewählt, dass für die freien Enden der Schienenelemente 334, 335, 366, 337 eine ausreichende elastische Positionsveränderlichkeit erhalten wird, die sicherstellt, dass die daran angeordneten Rückhalteelemente 338, 339, 340, 341 beim Retrahieren des geteilten Sternums mit den angrenzenden Rippen an die herrschenden Widerstandskräfte angepasste Kräfte ausüben können. Bei jeder dieser Varianten sind die Schienenelemente unabhängig voneinander in der Spreizebene verschwenkbar gehalten. Auch können die Schienenelemente 334, 335, 336, 337 eine unterschiedliche Länge aufweisen.

Darüber hinaus kann es vorteilhaft sein, wenn die Biegesteifigkeit der Schienenelemente sich entlang der Längsrichtung ändert, um so in jedem Abstand von den Gabelzinken 330, 331, 332, 333 eine den Rippen angepasste Federsteifigkeit zu realisieren. nenelemente sich entlang der Längsrichtung ändert, um so in jedem Abstand.

Ferner kann bei einer Variante auch vorgesehen sein, dass ein Anschlag die maximale Auslenkung eines Schienenelements in der Spreizebene begrenzt.

Vorzugsweise sind die Rückhalteelemente 338, 339, 340, 341 in Längsrichtung der Schienenelemente 334, 335, 336, 337 verschieblich angeordnet. Dadurch kann die Retraktionsvorrichtung zusätzlich an die Gegebenheiten einer aktuellen Operationssituation angepasst werden.

Die Rückhalteelemente 338, 339, 340, 341 sind im Wesentlichen so ausgebildet wie die bereits im Zusammenhang mit der Figur 1 beschriebenen Rückhalteelemente 50, 51, 52, 53, so dass auf die dort im Detail gegebene Beschreibung verwiesen werden kann.

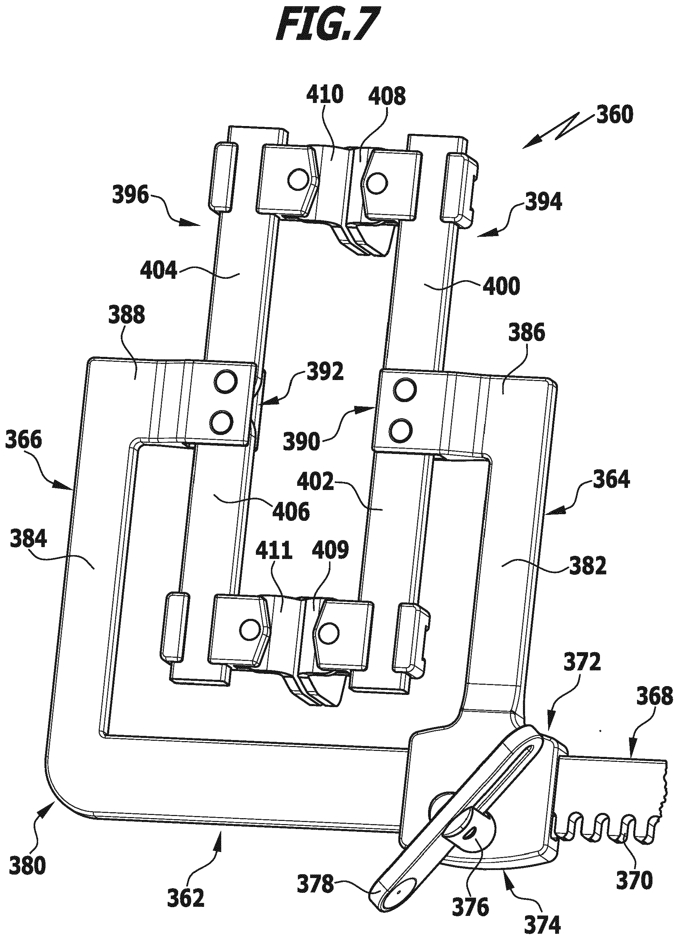

Die Figur 7 zeigt eine weitere Ausführungsform einer Retraktionsvorrichtung 360 mit einer Haltevorrichtung 362, an der einseitig zwei Spreizarme 364, 366 gehalten sind. Die Haltevorrichtung 362 umfasst eine im Wesentlichen gerade ausgebildete Schiene 368 mit einem im Wesentlichen rechteckigen Querschnitt, an dem an einer der Schmalseiten eine Zahnreihe 370 ausgebildet ist.

Der Spreizarm 364 ist entlang der Längsrichtung der Schiene 368 verfahrbar, so dass der Spreizarm 364 dem anderen Spreizarm 366 angenähert oder von dem Spreizarm 366 weg bewegt werden kann. Die beiden Spreizarme 364, 366 sind parallel zueinander ausgerichtet und definieren die Spreizebene der erfindungsgemäßen Retraktionsvorrichtung 360.

Der Spreizarm 364 ist mit einer Haltevorrichtung 372 an der Schiene 368 mittels eines Lagerelements 372 verfahrbar gehalten, wobei das Lagerelement 372 einen Antriebsmechanismus 374 umfasst, der einen Drehkörper (im Einzelnen nicht dargestellt), beispielsweise in Form eines Zapfenrades oder eines Zahnrades, umfasst, welcher auf einer Antriebswelle 376 drehfest montiert ist. Die Antriebswelle 376 steht oben aus dem Lagerelement 372 heraus und ist an ihrem freien Ende mit einem Griffteil 378 bestückt.

Mit Hilfe des Griffelements 378 lässt sich die Antriebswelle 376 und damit der Drehkörper rotieren, so dass daraus resultierend das Lagerelement 372 und der daran gehaltene Spreizarm 364 in Längsrichtung der Schiene 368 bewegt werden können. Vorzugsweise ist der Drehkörper der Antriebsvorrichtung 374 so ausgestaltet, dass sich eine selbsthemmende Verbindung zwischen dem Drehkörper und der Zahnreihe 370 der Schiene 368 ergibt.

Der Spreizarm 366 ist mit einem Ende an ein Ende 380 der Haltevorrichtung 362 rechtwinklig angeformt.

Die beiden Spreizarme 364, 366 sind zweiteilig ausgebildet, wobei ein erster Abschnitt 382, 384 an der Haltevorrichtung 362 in einem rechten Winkel gehalten ist. An ihrem freien, der Haltevorrichtung 362 entgegen gesetzten Ende tragen die beiden ersten Abschnitte 382, 384 gegeneinander abgewinkelte Bereiche 386, 388, welche ebenfalls in der Spreizebene der Retraktionsvorrichtung 360 angeordnet sind. Am freien Ende der abgewinkelten Bereiche 386, 388 sind Lagerstellen 390, 392 ausgebildet.

Die zweiten Abschnitte 394, 396 der Spreizarme 364, 366 sind als paarweise Schienenelemente 400, 402 bzw. 404, 406 ausgebildet, welche jeweils mit einem ihrer Enden verschwenkbar an den Lagerstellen 390, 392 gehalten sind. Die Lagerung der Schienenelemente geschieht vorzugsweise federnd, insbesondere mittels Zug-, Druck- oder Torsionsfedern (nicht dargestellt), wobei die Federkonstanten der einzelnen Schienenelemente unterschiedlich gewählt sein können. Durch die federnde Lagerung werden die Schienenelemente 400, 402, 404, 406 in einer im Wesentlichen parallelen Ausrichtung zum ersten Spreizarmabschnitt 382, 384 gehalten, bevor die Retraktionsbewegung einsetzt.

Auch können die Schienenelemente 400, 402, 404, 406 unterschiedliche Längen aufweisen.

An ihren freien Enden tragen die Schienenelemente 400, 402, 404, 406 Rückhalteelemente 408, 409, 410, 411, die ähnlich wie die im Zusammenhang mit Figur 1 schon beschriebenen Rückhalteelemente 50, 51, 52, 53 ausgebildet sind, so dass auf die dort gegebene detaillierte Beschreibung dieser Rückhalteelemente verwiesen werden darf.

Die Rückhalteelemente 408, 409, 410, 411 sind vorzugsweise entlang der Längsrichtung der geraden Schienenelemente 400, 402, 404, 406 angeordnet und lassen so eine auf den jeweiligen Applikationsfall angepasste Positionierung zu.

Aufgrund der elastisch schwenkbaren Lagerung der Schienenelemente 400, 402, 404, 406 wird beim Retrahieren der Spreizarme 364, 366 die Retraktionskraft adäquat auf die jeweiligen Abschnitte des geteilten Sternums und die daran angeschlossenen Rippenbögen ausgeübt.

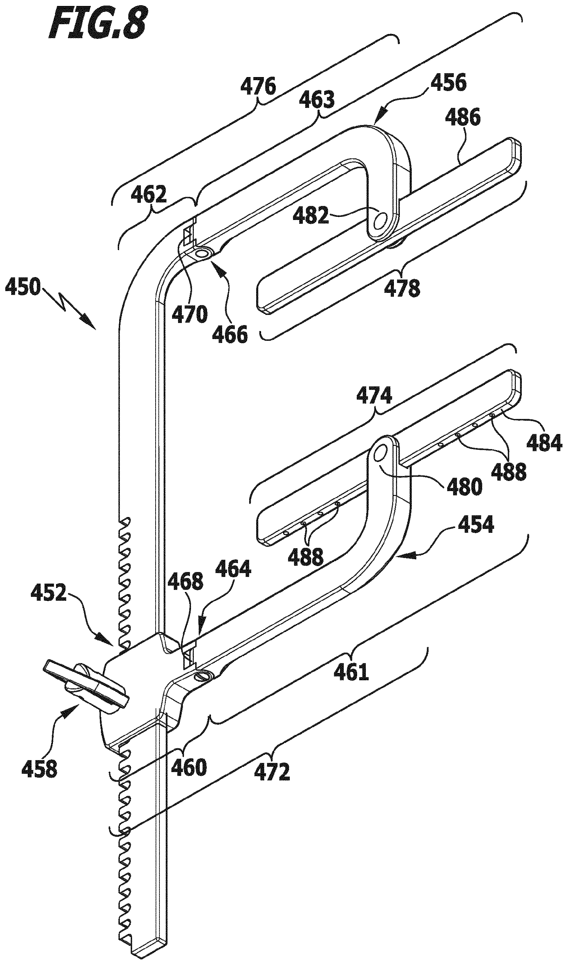

In Figur 8 ist eine chirurgische Retraktionsvorrichtung 450 dargestellt, in der die Prinzipien der vorliegenden Erfindung ebenfalls zur Anwendung kommen.

An einer Haltevorrichtung 452 sind zwei Spreizarme 454, 456 gehalten, wobei der Spreizarm 456 einstückig an der Haltevorrichtung 452 angeformt ist, während der Spreizarm 454 über einen Antriebsmechanismus 458 an der Haltevorrichtung 452 verschieblich gehalten ist.

Die Spreizarme 454 und 456 sind jeweils in zwei Segmente 460, 461 bzw. 462, 463 unterteilt, wovon das jeweils erste Segment 460 bzw. 462 in einer festen, im Wesentlichen unveränderlichen Ausrichtung zur Spreizebene der Retraktionsvorrichtung 450 an der Haltevorrichtung 452 gehalten ist.

Die beiden Segmente 460, 461 bzw. 462, 463 sind jeweils über ein Gelenk 464, 466 schwenkbar miteinander verbunden, wobei über einen Anschlag 468, 470 der Schwenkwinkel der zweiten Segmente 461, 463 zu dem jeweils zugeordneten ersten Segment 460, 462 beschränkt ist, so dass beispielsweise in der Figur 8 gezeigten Ausführungsform die zweiten Segmente 461, 463 nur aus der gezeigten Ausrichtung nach unten verschwenkbar sind, so dass die Haltevorrichtung 452, die typischerweise seitlich entlang des Rumpfs des Patienten zu liegen kommt, nach unten verschwenkt werden kann, so dass der Platzbedarf der Retraktionsvorrichtung 450 in der Spreizebene gesehen vermindert wird.

Bei der Retraktionsvorrichtung der Figur 8 ist darüber hinaus noch ein anderes Konzept der vorliegenden Erfindung realisiert, nämlich die Unterteilung der Spreizarme 454, 456 in einen ersten und einen zweiten Abschnitt, wovon der jeweils erste Abschnitt 472, 476 direkt an der Haltevorrichtung 452 gehalten ist und an seinem jeweiligen freien Ende 480 bzw. 482 den zweiten Abschnitt 474 bzw. 478 in der Spreizebene schwenkbar hält. Die freien Enden 480, 482 der ersten Abschnitte sind jeweils gegenüber der Längsrichtung der Spreizarme 454 bzw. 456 gegeneinander in der Spreizebene abgewinkelt ausgebildet.

Bezüglich der Unterteilung in erste und zweite Abschnitte der Spreizarme 454 und 456 entspricht das Konzept im Wesentlichen dem in Figur 5 gezeigten und dort auch beschriebenen Konzept, so dass für weitere Einzelheiten auch auf die Beschreibung der Figur 5 verwiesen werden kann.

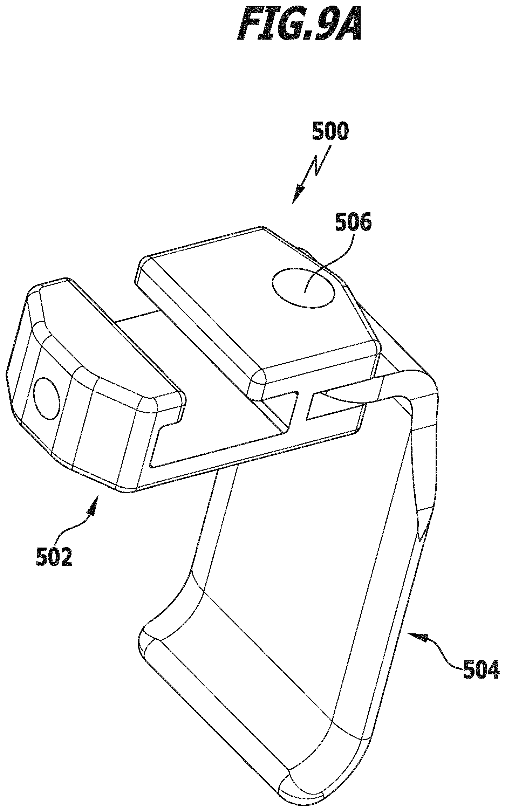

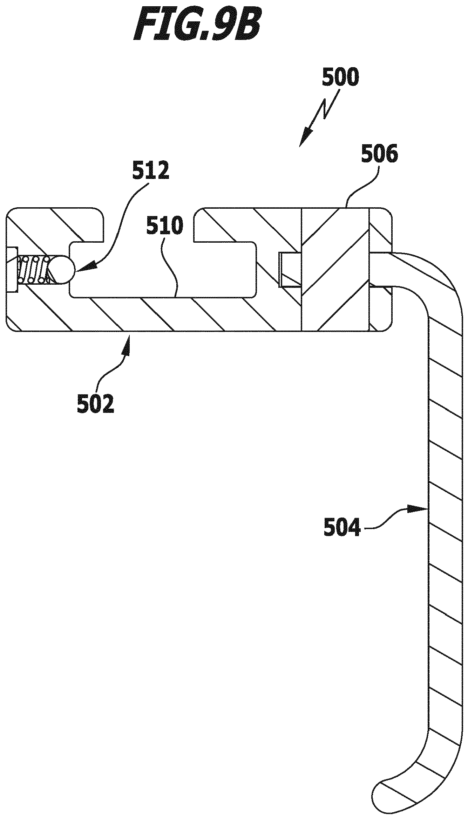

Darüber hinaus ist im Rahmen der Ausführungsformen der Figur 8 vorgesehen, dass die Rückhalteelemente an verschiedenen, definierten Positionen entlang der zweiten Abschnitte 474, 478 der Spreizarme montiert werden können. Zu diesem Zweck weisen die zweiten Abschnitte 474, 478 auf ihrer jeweiligen Rückseite 484 bzw. 486 Ausnehmungen 488 als Rastpositionen auf, in die Rückhalteelemente, wie sie im Folgenden im Zusammenhang mit den Figuren 9A und 9B im Einzelnen beschrieben werden, rastend fixiert werden können.

Aufgrund der Ausbildung der Rastpositionen 488 an den zweiten Abschnitten 474 und 478 der beiden Spreizarme 454, 456 ist es mit einfachen Mitteln möglich, gegenüber liegend aufeinander ausgerichtete Rückhalteelemente auf den zweiten Abschnitten 474, 478 der beiden Spreizarme zu platzieren.

Bei einer Retraktion mit der chirurgischen Retraktionsvorrichtung 450 findet eine Anpassung der auf das geteilte Sternum ausgeübten Kräfte wieder selbsttätig statt, indem sich die zweiten Abschnitte 474, 478 entsprechend den auftretenden Kräften in der Spreizebene verschwenken lassen und so angepasst die Retraktionskräfte auf das geteilte Sternum ausüben.

Die Figuren 9A und 9B zeigen eine spezielle Ausführungsform eines Rückhalteelementes 500, welches zweiteilig ausgebildet ist und ein Lagerelement 502 sowie eine Rückhaltefläche 504 umfasst.

Die Rückhaltefläche 504 ist an dem Lagerelement 502 um eine Achse 506, senkrecht zur Spreizebene, drehbar gelagert.

Das Lagerelement 502 weist einen im Wesentlichen rechteckigen Querschnitt auf, an dem auf einer Seite die Rückhaltefläche 504 mit der Drehachse 506 angeordnet ist. In dem rechteckigen Querschnitt des Lagerelements 502 ist eine rechteckige Ausnehmung 510 ausgebildet, welche in ihrem Querschnitt im Wesentlichen dem Querschnitt des zweiten Abschnitts 474 bzw. 478 der Retraktionsvorrichtung 450 entspricht.

Die Ausnehmung 510 nimmt den Querschnitt der zweiten Abschnitte 474, 478 der Spreizarme 454 bzw. 456 gleitend auf und kann aufgrund eines an der der Lagerung 506 gegenüber liegenden Schmalseite des Lageteils 502 angeordneten federbelasteten Kugeldruckstücks 512 an den vorgegebenen Rastpositionen 488 der zweiten Abschnitte 474 bzw. 478 platziert werden.

## Patentansprüche

1. Chirurgische Retraktionsvorrichtung (140,200,240,450), insbesondere zum Spreizen eines durchtrennten Sternums, mit einer Haltevorrichtung (142,202,242,452), zwei Spreizarmen (144,146,204,206,244,246,454,456),
wobei die Spreizarme mit jeweils einem Ende an der Haltevorrichtung in einem in einer Spreizebene in einer Retraktionsrichtung veränderlichen Abstand voneinander gehalten sind, sowie mindestens zwei an jedem der Spreizarme angeordneten Rückhalteelementen (180,181,182,183,222,223,224,225,280,281,282,283) wobei mindestens eines der Rückhalteelemente gegenüber dem an der Haltevorrichtung gehaltenen Ende des Spreizarms in der Retraktionsrichtung positionsveränderlich gelagert ist,
wobei beide Spreizarme in zwei Abschnitte unterteilt sind, wobei die ersten Abschnitte der Spreizarme in einem in Retraktionsrichtung in der Spreizebene veränderlichen Abstand im Wesentlichen starr an der Haltevorrichtung gehalten sind und der jeweils zweite Abschnitt der Spreizarme am ersten Abschnitt um eine Schwenkachse senkrecht zur Spreizebene schwenkbar gehalten ist,
wobei an dem zweiten Abschnitt der Spreizarme jeweils zwei Rückhalteelemente angeordnet sind, wobei die Rückhalteelemente beidseits der Schwenkachse angeordnet sind, **dadurch gekennzeichnet, dass** die Rückhalteelemente entlang der Längsrichtung des zweiten Abschnitts der jeweiligen Spreizarme verstellbar angeordnet sind.

2. Chirurgische Retraktionsvorrichtung nach Anspruch 1, **dadurch gekennzeichnet, dass** das mindestens eine Rückhalteelement in seiner Position gegenüber dem Spreizarm in Retraktionsrichtung federelastisch gelagert ist, und/oder
dass der erste Abschnitt eines unterteilten Spreizarms eine Länge aufweist, die größer ist als die Hälfte der gesamten Längsausdehnung des Spreizarms.

3. Chirurgische Retraktionsvorrichtung nach Anspruch 1 oder 2, **dadurch gekennzeichnet, dass** der zweite Abschnitt des Spreizarms federelastisch in einer Ausgangsstellung mit vorzugsweise koaxialer Ausrichtung zum ersten Abschnitt gehalten ist und/oder die Verschwenkbarkeit des zweiten Abschnitts bezüglich des ersten Abschnitts mittels eines Anschlags begrenzt ist und/oder der zweite Abschnitt in einer gegenüber dem ersten Abschnitt verschwenkten Position arretierbar ist und/oder der zweite Abschnitt mittels eines Antriebs gegenüber dem ersten Abschnitt verschwenkbar ist, wobei der Antrieb vorzugsweise selbsthemmend ausgebildet ist.

4. Chirurgische Retraktionsvorrichtung nach einem der Ansprüche 1 bis 3, **dadurch gekennzeichnet, dass** der erste Abschnitt im Wesentlichen linear ausgebildet ist, oder
der erste Abschnitt an seinem der Haltevorrichtung abgewandten Ende in der Spreizebene abgewinkelt ausgebildet ist, wobei der abgewinkelte Bereich in Richtung zum anderen Spreizarm weist und wobei die Schwenkachse in dem abgewinkelten Bereich des ersten Abschnitts angeordnet ist.

5. Chirurgische Retraktionsvorrichtung nach einem der Ansprüche 1 bis 4, **dadurch gekennzeichnet, dass** die Schwenkachse für den zweiten Abschnitt von einem Lagerelement gebildet wird, welches verschieblich an dem ersten Abschnitt gehalten ist.

6. Chirurgische Retraktionsvorrichtung nach einem der Ansprüche 1 bis 5, **dadurch gekennzeichnet, dass** der zweite Abschnitt an einem biegeelastischen Bereich des ersten Abschnitts gehalten ist.

7. Chirurgische Retraktionsvorrichtung nach einem der Ansprüche 4 bis 6, **dadurch gekennzeichnet, dass** der zweite Abschnitt in der Spreizebene biegeelastisch verformbar ausgebildet ist.

8. Chirurgische Retraktionsvorrichtung nach einem der Ansprüche 4 bis 7, **dadurch gekennzeichnet, dass** der zweite Abschnitt in zwei Längselemente unterteilt ist, die in entgegengesetzte Richtungen vom ersten Abschnitt abstehend angeordnet sind.

9. Chirurgische Retraktionsvorrichtung nach einem der Ansprüche 1 bis 8, **dadurch gekennzeichnet,**
**dass** die Rückhalteelemente in Laufrichtung der beiden Spreizarme zueinander versetzt angeordnet sind.

10. Chirurgische Retraktionsvorrichtung nach einem der Ansprüche 1 bis 9, **dadurch gekennzeichnet, dass** mindestens eines der Rückhalteelemente zweiteilig ausgebildet ist, wobei ein erstes Teil des Rückhalteelements ein Lagerteil bildet und am Spreizarm gehalten ist und das zweite Teil eine Rückhaltefläche bildet, welche beweglich, insbesondere um eine zur Spreizebene senkrechte und/oder parallele Achse schwenkbar, an dem ersten Teil gelagert ist und/oder
dass mindestens eines der Rückhalteelemente eine Rückhaltefläche aufweist, welche in zwei Abschnitte unterteilt ist, wobei ein erster Abschnitt an dem Spreizarm, ggf. über ein Lagerteil schwenkbar, gehalten ist und der zweite Abschnitt am ersten Abschnitt um eine parallel zur Längsrichtung des Spreizarms ausgerichtete Achse schwenkbar gelagert ist.

11. Chirurgische Retraktionsvorrichtung nach einem der Ansprüche 1 bis 10, **dadurch gekennzeichnet, dass** mindestens eines der Rückhalteelemente eine Rückhaltefläche aufweist, welche in zwei Abschnitte unterteilt ist, wobei ein erster Abschnitt an dem Spreizarm, ggf. über ein Lagerteil schwenkbar, gehalten ist und wobei der zweite Abschnitt in veränderlichem Abstand zur Spreizebene an dem ersten Abschnitt, ggf. um eine Achse parallel zur Längsrichtung des Spreizarms schwenkbar, gehalten ist.

12. Chirurgische Retraktionsvorrichtung nach einem der Ansprüche 1 bis 11, **dadurch gekennzeichnet, dass** mindestens eines der Rückhalteelemente eine Rückhaltefläche für das Sternum aufweist, welche an ihrem freien Ende abgekröpft und/oder stegartig verjüngt ausgebildet ist.

13. Chirurgische Retraktionsvorrichtung nach einem der Ansprüche 1 bis 12, **dadurch gekennzeichnet, dass** die Spreizarme in zwei Segmente unterteilt sind, wobei ein erstes Segment (460, 462) an der Haltevorrichtung bezüglich der Spreizebene in einer im Wesentlichen unveränderlichen Ausrichtung gehalten ist und wobei ein zweites Segment (461, 463) am freien Ende des ersten Segments in einer Ebene senkrecht zur Spreizebene verschwenkbar gehalten ist.

14. Chirurgische Retraktionsvorrichtung nach Anspruch 13, **dadurch gekennzeichnet, dass** der Schwenkwinkel der zweiten Segmente begrenzt ist, vorzugsweise derart, dass eine Schwenkbewegung des zweiten Segments nur in einer Richtung aus der Spreizebene heraus möglich ist.

15. Chirurgische Retraktionsvorrichtung nach Anspruch 13 oder 14, **dadurch gekennzeichnet, dass** die Länge der ersten Segmente der Spreizarme kleiner gewählt ist als die Länge der zweiten Segmente, wobei die Länge der ersten Segmente vorzugsweise ca. 30 % oder weniger der Gesamtlänge der Spreizarme beträgt.

## Claims

1. Surgical retractor (140, 200, 240, 450), in particular for spreading a severed sternum, comprising a holding device (142, 202, 242, 452), two spreader arms (144, 146, 204, 206, 244, 246, 454, 456), wherein the spreader arms in each case are held at one end thereof on the holding device at a distance from one another that is variable in a retraction direction in a spreading plane, and at least two retaining elements (180, 181, 182, 183, 222, 223, 224, 225, 280, 281, 282, 283) arranged on each of the spreader arms, wherein at least one of the retaining elements is supported for variable positioning in the retraction direction relative to the end of the spreader arm held on the holding device,
wherein both spreader arms are divided into two sections, wherein the first sections of the spreader arms are held substantially rigidly on the holding device at a variable distance in the retraction direction in the spreading plane and wherein the second section of the spreader arms in each case is held on the first section for pivotal movement about a pivot axis perpendicular to the spreading plane,
wherein the second section of the spreader arms in each case has two retaining elements arranged thereon, wherein the retaining elements are arranged on both sides of the pivot axis, **characterized in that**
the retaining elements are arranged for adjustment along the longitudinal direction of the second section of the respective spreader arms.

2. Surgical retractor in accordance with claim 1, **characterized in that** the at least one retaining element is supported resiliently in its position relative to the spreader arm in the retraction direction, and/or **in that**
the first section of a divided spreader arm has a length greater than half of the total longitudinal extent of the spreader arm.

3. Surgical retractor in accordance with claim 1 or 2, **characterized in that** the second section of the spreader arm is resiliently held in a starting position with a preferably coaxial orientation relative to the first section, and/or the pivotability of the second section relative to the first section is limited by a stop, and/or the second section is lockable in a position pivoted relative to the first section, and/or the second section is pivotable relative to the first section by way of a drive, preferably wherein the drive is of self-locking configuration.

4. Surgical retractor in accordance with any one of claims 1 to 3, **characterized in that** the first section is of substantially linear configuration, or **in that**
the first section, at an end thereof facing away from the holding device, is of angled configuration in the spreading plane, wherein the angled portion points in a direction towards the other spreader arm and wherein the pivot axis is arranged in the angled portion of the first section.

5. Surgical retractor in accordance with any one of claims 1 to 4, **characterized in that** the pivot axis for the second section is formed by a bearing element which is held for displacement on the first section.

6. Surgical retractor in accordance with any one of claims 1 to 5, **characterized in that** the second section is held at a flexurally elastic portion of the first section.

7. Surgical retractor in accordance with any one of claims 4 to 6, **characterized in that** the second section is configured for flexurally elastic deformation in the spreading plane.

8. Surgical retractor in accordance with any one of claims 4 to 7, **characterized in that** the second section is divided into two longitudinal elements which are arranged such that they project in opposite directions from the first section.

9. Surgical retractor in accordance with any one of claims 1 to 8, **characterized in that** the retaining elements are arranged in offset relation to one another in the longitudinal direction of the two spreader arms.

10. Surgical retractor in accordance with any one of claims 1 to 9, **characterized in that** at least one of the retaining elements is configured in two parts, wherein a first part of the retaining element forms a bearing part and is held on the spreader arm and the second part forms a retaining surface which is supported on the first part for movement, in particular for pivotal movement about an axis perpendicular and/or parallel to the spreading plane, and/or **in that**
at least one of the retaining elements has a retaining surface which is divided into two sections, wherein a first section is held on the spreader arm, optionally for pivotal movement via a bearing part, and wherein the second section is supported on the first section for pivotal movement about an axis oriented parallel to the longitudinal direction of the spreader arm.

11. Surgical retractor in accordance with any one of claims 1 to 10, **characterized in that** at least one of the retaining elements has a retaining surface which is divided into two sections, wherein a first section is held on the spreading arm, optionally for pivotal movement via a bearing part, and wherein the second section is held at a variable distance from the spreading plane on the first section, optionally for pivotal movement about an axis parallel to the longitudinal direction of the spreader arm.

12. Surgical retractor in accordance with any one of claims 1 to 11, **characterized in that** at least one of the retaining elements has a retaining surface for the sternum, which retaining surface has, at its free end, an offset configuration and/or a configuration tapering to a web-like shape.

13. Surgical retractor in accordance with any one of claims 1 to 12, **characterized in that** the two spreader arms are divided into two segments, wherein a first segment (460, 462) is held on the holding device in a substantially invariable orientation relative to the spreading plane and wherein a second segment (461, 463) is held at the free end of the first segment for pivotal movement in a plane perpendicular to the spreading plane.

14. Surgical retractor in accordance with claim 13, **characterized in that** the pivot angle of the second segments is limited, preferably in such a manner that a pivotal movement of the second segment is possible only in a direction out of the spreading plane.

15. Surgical retractor in accordance with claim 13 or 14, **characterized in that** the length of the first segments of the spreader arms is selected to be smaller than the length of the second segments, preferably wherein the length of the first segments is approximately 30 % or less of the overall length of the spreader arms.

## Revendications

1. Dispositif de rétraction chirurgical (140, 200, 240, 450), notamment pour assurer un écartement d'un sternum ayant été sectionné, comprenant un dispositif de maintien (142, 202, 242, 452), deux bras écarteurs (144, 146, 204, 206, 244, 246, 454, 456), les bras écarteurs étant tenus chacun par une extrémité sur le dispositif de maintien, à une distance l'un de l'autre, qui est variable dans une direction de rétraction dans un plan d'écartement, ainsi qu'au moins deux éléments de retenue (180, 181, 182, 183, 222, 223, 224, 225, 280, 281, 282, 283) agencés sur chacun des bras écarteurs, l'un au moins des éléments de retenue étant monté en position variable dans la direction de rétraction par rapport à l'extrémité du bras écarteur, qui est tenue sur le dispositif de maintien,
dispositif de rétraction,
dans lequel les deux bras écarteurs sont subdivisés en deux tronçons, les premiers tronçons des bras écarteurs étant maintenus de manière sensiblement rigide sur le dispositif de maintien selon une distance d'espacement variable dans la direction de rétraction dans le plan d'écartement, et le deuxième tronçon respectif des bras écarteurs étant maintenu sur le premier tronçon de manière à pouvoir pivoter autour d'un axe de pivotement perpendiculaire au plan d'écartement, et
dans lequel sur le deuxième tronçon des bras écarteurs sont agencés respectivement deux éléments de retenue, les éléments de retenue étant agencés de part et d'autre de l'axe de pivotement,
**caractérisé en ce que** les éléments de retenue sont agencés de manière réglable le long de la direction longitudinale du deuxième tronçon des bras écarteurs respectifs.

2. Dispositif de rétraction chirurgical selon la revendication 1, **caractérisé en ce que** ledit au moins un élément de retenue est monté de manière élastique dans la direction de rétraction dans sa position par rapport au bras écarteur, et/ou
**en ce que** le premier tronçon d'un bras écarteur subdivisé présente une longueur qui est supérieure à la moitié de l'étendue longitudinale totale du bras écarteur.

3. Dispositif de rétraction chirurgical selon la revendication 1 ou la revendication 2, **caractérisé en ce que** le deuxième tronçon du bras écarteur est maintenu de manière élastique dans une position initiale avec de préférence une orientation coaxiale au premier tronçon, et/ou la possibilité de pivotement du deuxième tronçon par rapport au premier tronçon est limitée au moyen d'une butée, et/ou le deuxième tronçon peut être bloqué dans une position pivotée par rapport au premier tronçon, et/ou le deuxième tronçon peut pivoter par rapport au premier tronçon au moyen d'un entraînement, l'entraînement étant de préférence réalisé autobloquant.

4. Dispositif de rétraction chirurgical selon l'une des revendications 1 à 3, **caractérisé en ce que** le premier tronçon est sensiblement de configuration linéaire, ou bien
le premier tronçon est, à son extrémité opposée à celle dirigée vers le dispositif de maintien, de configuration coudée dans le plan d'écartement, la zone coudée étant dirigée vers l'autre bras écarteur, et l'axe de pivotement étant agencé dans la zone coudée du premier tronçon.

5. Dispositif de rétraction chirurgical selon l'une des revendications 1 à 4, **caractérisé en ce que** l'axe de pivotement pour le deuxième tronçon est formé par un élément de palier, qui est maintenu de manière coulissante sur le premier tronçon.

6. Dispositif de rétraction chirurgical selon l'une des revendications 1 à 5, **caractérisé en ce que** le deuxième tronçon est maintenu sur une zone élastiquement flexible du premier tronçon.

7. Dispositif de rétraction chirurgical selon l'une des revendications 4 à 6, **caractérisé en ce que** le deuxième tronçon est de configuration élastiquement déformable dans le plan d'écartement.

8. Dispositif de rétraction chirurgical selon l'une des revendications 4 à 7, **caractérisé en ce que** le deuxième tronçon est subdivisé en deux éléments longitudinaux, qui sont agencés en s'éloignant du premier tronçon selon des directions opposées.

9. Dispositif de rétraction chirurgical selon l'une des revendications 1 à 8, **caractérisé en ce que** les éléments de retenue sont agencés de manière mutuellement décalée dans la direction longitudinale des deux bras écarteurs.

10. Dispositif de rétraction chirurgical selon l'une des revendications 1 à 9, **caractérisé en ce qu'**au moins l'un des éléments de retenue est réalisé en deux parties, une première partie de l'élément de retenue formant une partie de montage et étant maintenue sur le bras écarteur, et la deuxième partie formant une surface de retenue, qui est montée mobile, notamment pivotante autour d'un axe perpendiculaire et/ou parallèle au plan d'écartement, sur la première partie, et/ou
**en ce qu'**au moins l'un des éléments de retenue présente une surface de retenue, qui est subdivisée en deux tronçons, un premier tronçon étant maintenu sur le bras écarteur, le cas échéant de manière pivotante par l'intermédiaire d'un élément de palier, et le deuxième tronçon étant monté sur le premier tronçon de manière pivotante autour d'un axe orienté parallèlement à la direction longitudinale du bras écarteur.

11. Dispositif de rétraction chirurgical selon l'une des revendications 1 à 10, **caractérisé en ce qu'**au moins l'un des éléments de retenue présente une surface de retenue, qui est subdivisée en deux tronçons, un premier tronçon étant maintenu sur le bras écarteur, le cas échéant de manière pivotante par l'intermédiaire d'un élément de palier, et le deuxième tronçon étant maintenu sur le premier tronçon à distance variable par rapport au plan d'écartement, le cas échéant de manière pivotante autour d'un axe parallèle à la direction longitudinale du bras écarteur.

12. Dispositif de rétraction chirurgical selon l'une des revendications 1 à 11, **caractérisé en ce que** l'un au moins des éléments de retenue présente une surface de retenue pour le sternum, qui, à son extrémité libre, est d'une réalisation coudée et/ou rétrécie en languette.

13. Dispositif de rétraction chirurgical selon l'une des revendications 1 à 12, **caractérisé en ce que** les bras écarteurs sont subdivisés en deux segments, un premier segment (460, 462) étant maintenu sur le dispositif de maintien, dans une orientation sensiblement invariable relativement au plan d'écartement, et un deuxième segment (461, 463) étant maintenu à l'extrémité libre du premier segment en pouvant pivoter dans un plan perpendiculaire au plan d'écartement.

14. Dispositif de rétraction chirurgical selon la revendication 13, **caractérisé en ce que** l'angle de pivotement des deuxièmes segments est limité, de préférence de manière telle, qu'un mouvement de pivotement du deuxième segment ne soit possible que dans une seule direction hors du plan d'écartement.

15. Dispositif de rétraction chirurgical selon la revendication 13 ou la revendication 14, **caractérisé en ce que** la longueur des premiers segments des bras écarteurs est choisie plus petite que la longueur des deuxièmes segments, la longueur des premiers segments valant de préférence environ 30 % de la longueur totale des bras écarteurs, ou moins.
